# EUROPEAN PATENT APPLICATION

(11) **EP 4 834 315 A1**
(43) Date of publication of application: **04.11.2026**
(21) Application number: 24911844.9
(22) Date of filing: 20.08.2024
(51) Int. Cl.: A61K 8/36, A61Q 1/00, C07C 53/126, C11D 17/06

(54) **SOAP COMPOSITE PARTICLES, METHOD FOR PRODUCING SOAP COMPOSITE PARTICLES, AND COSMETIC**

(30) Priority: 26.12.2023 JP 2023219174
(71) Applicant: Tomoegawa Corporation, Tokyo 104-8335 (JP)
(72) Inventor: IKEYA Hirotoshi, Shizuoka-shi, Shizuoka 421-0192 (JP)
(74) Representative: V.O.
(86) International application number: PCT/JP2024/029436
(87) International publication number: WO 2025/141949

(57) **Abstract**

The present invention aims to provide soap composite particles that can achieve both improved skin feel and sustained functionality of the soap composite particles, a method for producing the soap composite particles, and a cosmetic containing the soap composite particles. The soap composite particles have core particles containing a saturated higher fatty acid salt and an inorganic oxide externally added to the core particle, wherein the total content of Li₂O, Na₂O, Al₂O₃, MgO, CaO, ZnO, BaO, TiO₂, SiO₂, Fe₃O₄, Fe₂O₃, and FeO in the core particle is 0 to 15 mass%, and the shape of the core particle is spherical with a circularity of 0.80 to 1.00.

## Description

### TECHNICAL FIELD

The present invention relates to soap composite particles, a method for producing soap composite particles, and a cosmetic.

### BACKGROUND ART

Patent Document 1 discloses a metal soap that can impart high dispersibility or coating properties to powder and improve the anti-caking properties, flowability, or tactile feel of the powder, and a method for producing the same. This metal soap is characterized by an internal incorporation rate A, defined as the proportion of the inorganic crystal nucleating agent incorporated into the interior of the metal soap particle, of 30% or more.

### Citation List

### Patent Document

Patent Document 1: Japanese Patent No. 6729546

### SUMMARY OF INVENTION

### Technical Problem

Patent Document 1 suggests that the inorganic crystal nucleating agent incorporated into the interior of the metal soap particles acts to make the particle shape uniform and the particle size distribution sharp, and as a result, the solid powder cosmetic preparations in the examples have a pleasant feel to the touch. However, Patent Document 1 does not describe or suggest how to achieve both an improvement in skin feel and the sustained functionality of the soap composite particles.

When a cosmetic contains a paraffin-based solvent such as isododecane, the sustained functionality of the metal soap particles may be affected compared to when a silicone-based solvent such as dimethicone is used.

The present invention has been made in consideration of the above circumstances, and aims to provide soap composite particles that can achieve both improved skin feel and sustained functionality of the soap composite particles, a method for producing the soap composite particles, and cosmetics containing the soap composite particles.

### Solution to Problem

A first aspect of the present invention provides soap composite particles comprising a core particle containing a saturated higher fatty acid salt and an inorganic oxide externally added to the core particle, wherein the total content of Li₂O, Na₂O, Al₂O₃, MgO, CaO, ZnO, BaO, TiO₂, SiO₂, Fe₃O₄, Fe₂O₃, and FeO in the core particle is 0 to 15 mass%, and the shape of the core particle is spherical with a circularity of 0.80 to 1.00.

A second aspect is the first aspect, wherein the saturated higher fatty acid salt has a carbon number in the range of 10 to 20.

A third aspect is the first or second aspect, wherein the metal forming the saturated higher fatty acid salt is at least one selected from Li, Na, Al, Mg, Ca, Zn, and Ba.

A fourth aspect is any one of the first to third aspects, wherein the amount of the inorganic oxide added externally is 0.01 to 15 parts by weight relative to 100 parts by weight of the core particles.

A fifth aspect is any one of the first to fourth aspects, wherein the inorganic oxide is one or more kind selected from zinc oxide, magnesium oxide, titanium dioxide, mica, talc, kaolin, sericite, silica, iron oxide, alumina, zirconia, and zeolite.

A sixth aspect is any one of the first to fifth aspects, wherein the inorganic oxide is subjected to a hydrophobic treatment.

A seventh aspect is to the sixth aspect, wherein the hydrophobic treatment is any one of a silane coupling treatment, a silicone oil treatment, a long-chain alkylsilane treatment, a fatty acid salt treatment, a long-chain alkane treatment, a long-chain alkene treatment, and a long-chain alkyne treatment.

An eighth aspect is any one of the first to seventh aspects, wherein the inorganic oxide has an average primary particle size of 0.01 to 1.0 µm.

A ninth aspect is any one of the first to eighth aspects, wherein the volume-based median diameter of the core particle is greater than 3 µm and less than 20 µm.

A tenth aspect is any one of the first to ninth aspects, comprising a pulverization step of pulverizing a solid of the saturated higher fatty acid salt, a spheroidization step of spheroidizing the particles obtained in the pulverization step, and an external addition step of externally adding an inorganic oxide to the core particles obtained in the spheroidization step.

An eleventh aspect is any one of the first to ninth aspects, comprising a pulverization step of pulverizing a solid of the saturated higher fatty acid salt, an external addition step of externally adding an inorganic oxide to the particles obtained in the pulverization step, and a spheroidization step of spheroidizing the particles obtained in the external addition step.

A twelfth aspect is a cosmetic characterized by comprising the soap composite particles according to any one of the first to ninth aspects.

### Advantageous Effects of Invention

According to the present invention, it is possible to provide soap composite particles that can achieve both improved skin feel and sustained functionality of the soap composite particles, a method for producing the soap composite particles, and a cosmetic containing the soap composite particles.

### DESCRIPTION OF EMBODIMENTS

The present invention will be described below using embodiments. However, these embodiments are specifically described to provide a better understanding of the gist of the invention, and unless otherwise specified, do not limit the present invention. Other embodiments in which those skilled in the art appropriately replace the configurations of the following embodiments are also included within the scope of the present invention.

The soap composite particle of the present embodiment has a core particle containing a saturated higher fatty acid salt and an inorganic oxide externally added to the core particle.

### <Saturated higher fatty acid salt>

The core particles contain a saturated higher fatty acid salt. The saturated higher fatty acid salt may be a salt of an alkali metal such as sodium or potassium, or a salt of a metal other than an alkali metal. The saturated higher fatty acid salt may be produced by a conventional method using saturated higher fatty acid or its alkali metal salt and inorganic metal salt as a raw material, or commercially available products may be used. The saturated higher fatty acid salt may be one kind or a mixture of two or more kinds.

The saturated higher fatty acid used in the saturated higher fatty acid salt is not particularly limited, but preferred examples include hexanoic acid, caproic acid (n-hexanoic acid), heptanoic acid, octanoic acid, caprylic acid (n-octanoic acid), nonanoic acid, decanoic acid, capric acid (n-decanoic acid), lauric acid, myristic acid, palmitic acid, stearic acid, arachic acid, behenic acid, and montanic acid.

Saturated higher fatty acids do not have double bonds (unsaturated bonds) in the hydrocarbon chain, making them highly chemically stable, less susceptible to deterioration by oxygen or ultraviolet rays, and more likely to maintain their excellent quality. The saturated higher fatty acid salt may be a branched chain, but is preferably a saturated higher fatty acid salt having two or less carbon atoms in the branched side chain, and more preferably a saturated higher fatty acid salt having the number of carbon atoms, one or less. Straight-chain saturated higher fatty acid salts are particularly preferred.

When the saturated higher fatty acid salt is a straight-chain saturated higher fatty acid salt, the hydrocarbon chain has no double bond (unsaturated bond) and no side chain, and therefore the structure is stable. In addition, the methyl groups at the molecular ends are neatly arranged on the surface of the soap composite particles, and because the melting point is high, it is difficult to become liquid at room temperature (5 to 35°C), making it easy to maintain its excellent quality.

The number of carbon atoms in the saturated higher fatty acid salt is not particularly limited, but may be, for example, within the range of 8 to 30, preferably within the range of 10 to 20, more preferably within the range of 10 to 18, and particularly preferably within the range of 10 to 16. When the number of carbon atoms is equal to or greater than the lower limit, the saturated higher fatty acid salt exhibits hydrophobicity and is less susceptible to the effects of moisture. When the number of carbon atoms is equal to or less than the upper limit, the molecules are easily aligned, the structure is stable, the melting point is high, and the shape of the core particle is stabilized. Furthermore, the sustainability of the inorganic oxide function is improved.

The metal salt used in the saturated higher fatty acid salt is not particularly limited, but examples thereof include alkali metal salts, alkaline earth metal salts, and transition metal salts. Examples of metals that form saturated higher fatty acid salts include one or more kind of Li, Na, Al, Mg, Ca, Zn, and Ba, preferably one or more kind of Zn, Mg, Ca, and Al, and more preferably one or more kind of Zn, Mg, and Ca. These compounds form stable structures in the soap itself. Among these, it is preferable to select a compound that has little effect on the human body and is insoluble in water.

In particular, in applications where the composition containing the soap composite particles is applied to the skin, it is preferable that the molecular ends of the saturated higher fatty acid salt form a uniform hydrophobic surface. This makes it less susceptible to the effects of moisture in the air and moisture in body fluids such as sweat, improving the feel on the skin. Compositions that are applied to the skin can be used in cosmetics, medicines, other pharmaceuticals, and the like.

The saturated higher fatty acid salt preferably does not contain any polar functional groups such as hydroxyl groups or epoxy groups, except for the carboxylate ion group (-COO⁻) at one end. This improves the hydrophobicity. When the general formula of the saturated higher fatty acid salt is expressed as (RCOO)ₘM, at least a part or all of the saturated higher aliphatic groups R are preferably non-cyclic alkyl groups represented by Cₙ₋₁H₂ₙ₋₁-, and are preferably linear alkyl groups represented by CH₃(CH₂)ₙ₋₂-. In the branched alkyl group, the branch is preferably located at a position away from the carboxylate ion group, and examples thereof include (CH₃)₂CH(CH₂)ₙ₋₄-. It preferably contains a non-cyclic compound represented by the general formula (Cₙ₋₁H₂ₙ₋₁COO)ₘM, and more preferably contains a linear compound represented by the general formula [CH₃(CH₂)ₙ₋₂COO]ₘM. In these general formulas, n represents the number of carbon atoms, and M represents a metal.

When the metal M in the saturated higher fatty acid salt is polyvalent, that is, when m is 2 or greater, the carbon number n of the m saturated higher fatty acids may be the same or different. When the carbon numbers n of the m saturated higher fatty acids are different from one another, the difference between the maximum carbon number and the minimum carbon number is preferably 4 or less, and more preferably 2 or less. In order for the molecular ends of the saturated higher fatty acid salt to form a more uniform hydrophobic surface, it is preferable that the number of carbon atoms of each saturated higher fatty acid constituting the saturated higher fatty acid salt is the same.

### <Core particle>

The core particles have a spherical shape with a circularity of 0.80 to 1.00. It is more preferable that the circularity of the core particles is 0.90 to 1.00. The saturated higher fatty acid salt in the core particles acts as a lubricant, making them roll easily without resistance. When the circularity of the core particles is equal to or greater than the lower limit, the particles tend to roll and slide without resistance. For example, when a composition containing soap complex particles is applied onto the skin using the pads of the fingers, the particles roll easily, allowing for a good effect on the skin. The theoretical maximum value of circularity is 1, which corresponds to the case where the particle image is a perfect circle.

The circularity can be calculated by analyzing a particle image, measuring the area A and perimeter C of the particle image, and using the following formula, where D is the diameter of a circle (equivalent circle diameter) equal to the area A of the particle image and π is the ratio of the circumference of the particle to its diameter. $\left(Circularity\right) = πD / C$

The circularity of particles may be measured using, for example, a flow particle image analyzer that measures particle shape, particle size distribution, particle number, etc. by two-dimensional image analysis of a particle group. Alternatively, the circularity may be calculated by observing the particle surface from multiple fields of view using an electron microscope and performing a stereoscopic image analysis.

In the soap composite particle of this embodiment, the total content of Li₂O, Na₂O, Al₂O₃, MgO, CaO, ZnO, BaO, TiO₂, SiO₂, Fe₃O₄, Fe₂O₃, and FeO in the core particle is 0 to 15 mass%. These metal oxides (hereinafter sometimes referred to as "specific metal oxides") may be derived from an inorganic oxide added externally to the core particles, may be derived from an inorganic oxide added internally to the core particles, or may be derived from a saturated higher fatty acid salt. The specific metal oxide may be different from the inorganic oxide added externally to the core particle, or may be different from the inorganic oxide added internally to the core particle.

When the core particle does not contain the specific metal oxide, it is preferable that no inorganic oxide is added internally to the core particle, or that an inorganic oxide different from the specific metal oxide is added to the core particle. In this case, the content (total content) of the specific metal oxide in the core particle may be 0% by weight, but the specific metal oxide may be mixed into the core particle as an impurity, or part of the inorganic oxide externally added to the core particle may be contained in the core particle.

When the core particle contains a specific metal oxide, the inorganic oxide added to the core particle preferably contains the specific metal oxide. In this case, the content (total content) of the specific metal oxide in the core particle is preferably 0.01 % by weight or more.

In particular, when the specific metal oxide is Al₂O₃, MgO, ZnO, TiO₂, SiO₂, Fe₃O₄, Fe₂O₃, or FeO and also serves as a functional inorganic oxide that is preferably added externally or internally to the core particles, the content is preferably 0.01% by weight or more. This allows the inorganic oxide to exert its functions and improves its sustainability.

The metals (Li, Na, Al, Mg, Ca, Zn, Ba, Ti, Si, Fe) contained in the specific metal oxide may be the same as the metals (preferably Li, Na, Al, Mg, Ca, Zn, Ba) that form the saturated higher fatty acid salt. When the content of the specific metal oxide in the core particles is equal to or less than the upper limit, the crystallinity of the saturated higher fatty acid salt is easily maintained. This makes it easier for the core particles to maintain their shape, improving the feel on the skin. When the metal contained in the specific metal oxide and the metal forming the saturated higher fatty acid salt are each selected from the group consisting of Li, Na, Al, Mg, Ca, Zn, and Ba, different metals may be selected.

The volume-based median diameter of the core particles is preferably more than 3 µm and less than 20 µm, more preferably more than 5 µm and less than 15 µm, and even more preferably more than 7 µm and less than 10 µm. If the volume-based median diameter of the core particles exceeds the lower limit, the particles have a small cohesive force and are less likely to form agglomerates. If the volume-based median diameter of the core particles is less than the upper limit, the surface area per unit weight increases, the exposed area of the inorganic oxide increases, and the sustainability of the inorganic oxide function improves.

In applications where a composition containing the soap composite particles is applied to the skin, if the volume-based median diameter of the core particles exceeds the lower limit, the composition spreads easily on the skin and provides a uniform skin feel when spread. When the volume-based median diameter of the core particles is less than the upper limit, the roughness caused by the particle size is less felt on the fingertips, improving the feel on the skin.

### <Inorganic oxides>

The soap composite particle of the embodiment has an inorganic oxide externally added to the core particle. As described above, the core particles are not particularly limited as long as they have a structure containing a saturated higher fatty acid salt, but inorganic oxides may be added internally to the core particles. The inorganic oxide added internally to the core particle may be embedded within the core particle. The inorganic oxide added externally to the outside of the core particle preferably covers at least a part of the outer surface of the core particle.

Examples of the inorganic oxide include inorganic particles of metal oxides, metal nitrides, silicates, sulfates, carbonates, phosphates, and the like. Specific examples of inorganic oxides include one or more kind of zinc oxide, magnesium oxide, titanium dioxide, mica, talc, kaolin, sericite, silica, iron oxide, alumina, zirconia, and zeolite.

The inorganic oxide particles are preferably functional particles. The functionality of the soap composite particles is preferably such that when they are used, the inorganic oxide particles act on an object together with the saturated higher fatty acid salt. Specific examples of functionality include, but are not limited to, ultraviolet reflectivity, sunscreen, hiding power as a pigment, anti-inflammatory properties against inflammation, X-ray absorption, solid lubricity, release properties, antibacterial properties, and electrical insulation properties.

The content of inorganic oxide in the soap composite particles (total amount of internally added and externally added) is preferably 3 to 80% by weight. When the total amount is equal to or greater than the lower limit, the exposed area of the inorganic oxide increases, improving the sustainability of the inorganic oxide function. When the total amount is equal to or less than the upper limit, the bond between the saturated higher fatty acid salt and the inorganic oxide is stabilized, and the uniformity of the soap composite particles is improved.

The content (internal addition amount) of the inorganic oxide in the core particles is preferably 5 to 75% by weight, more preferably 5 to 10% by weight. When the amount of internal addition is equal to or greater than the lower limit, the exposed area of the inorganic oxide increases, improving the sustainability of the inorganic oxide function. When the amount of the internal addition is equal to or less than the upper limit, the saturated higher fatty acid salt and the inorganic oxide are easily kneaded together, and the uniformity of the core particles is improved.

The amount of the inorganic oxide added externally relative to 100 parts by weight of the core particles is preferably 0.01 to 15 parts by weight, more preferably 0.05 to 8.0 parts by weight. When the amount of the external addition is equal to or greater than the lower limit, the amount of inorganic oxide present on the surface of the core particle increases, improving the sustainability of the function of the inorganic oxide. When the amount of external addition is equal to or less than the upper limit, the lubricity and feel to the touch that are characteristic of saturated higher fatty acid salts become more dominant.

The inorganic oxide added to the outside of the core particle may be the same type of inorganic oxide as the inorganic oxide added internally to the core particle, or may be a different inorganic oxide. The inorganic oxide to be added internally to the core particle may be one kind or two or more kinds. When two or more inorganic oxides are added internally to the core particles, the amount of the internally added inorganic oxides may be the total amount of the two or more kinds of inorganic oxides. The inorganic oxide to be externally added to the core particles may be one kind or two or more kinds. When two or more kinds of inorganic oxides are added externally to the core particles, the amount of the externally added inorganic oxides may be the total amount of the two or more kinds of the inorganic oxides.

The inorganic oxide externally added to the core particles may function as a fluidity imparting agent. In this case, the fluidity imparting agent exerts a bearing effect, increasing the fluidity of the soap composite particles and improving the feel on the skin. As the inorganic oxide to be externally added to the core particles, particles that function as a fluidity imparting agent and particles that have other functions may be used in combination.

The inorganic oxide added externally or internally to the core particles may be coated, for example, may be subjected to a hydrophobic treatment. The hydrophobic treatment is not particularly limited, but examples thereof include silane coupling treatment, silicone oil treatment, long-chain alkylsilane treatment, fatty acid salt treatment, long-chain alkane treatment, long-chain alkene treatment, and long-chain alkyne treatment. In the silicone oil treatment, modified silicone oil may be used.

In the case of silane coupling treatment or silicone oil treatment, siloxane bonds are formed and fixed on the surface of the inorganic oxide by hydrolysis of the silane coupling agent or silicone oil and a dehydration condensation reaction, and the inorganic oxide is made hydrophobic. Therefore, inorganic oxides have high dispersibility even when kneaded into soap made of a hydrophobic higher saturated fatty acid salt at high temperatures.

In the case of the fatty acid salt treatment, the composition of the fatty acid salt is the same as or similar to the composition of the higher saturated fatty acid salt used in soap. Therefore, inorganic oxides that have been surface-treated with a fatty acid salt have high dispersibility in the soap into which they are kneaded, and have high adhesion to the soap. When the fatty acid salt used in the fatty acid salt treatment corresponds to the above-mentioned higher saturated fatty acid salt, the mass of the higher saturated fatty acid salt may be excluded from the mass of the inorganic oxide and added to the mass of the higher saturated fatty acid salt.

In the case of long-chain alkylsilane treatment, long-chain alkane treatment, long-chain alkene treatment, and long-chain alkyne treatment, the inorganic oxide can be made hydrophobic by using a corresponding long-chain aliphatic compound. The chain length of these long-chain aliphatic compounds is not particularly limited, but may be, for example, 8 to 30 carbon atoms.

The inorganic oxide to be added externally and/or internally to the core particles preferably has an average primary particle size of 0.01 to 1.0 µm. When the average primary particle size of the inorganic oxide is equal to or greater than the lower limit, the inorganic oxide particles are less likely to aggregate and are more likely to disperse. In particular, when such inorganic oxides are externally added, the soap composite particles have a good feel on the skin. When the average primary particle size of the inorganic oxide is equal to or less than the upper limit, the inorganic oxide is easily added internally to the core particles, and even when added externally, it is difficult for the inorganic oxide to fall off from the soap composite particles. In particular, when such an inorganic oxide is externally added, the bumpy feeling of the inorganic oxide (surface roughness caused by the particles) is reduced, and the feel of the soap composite particles on the skin is improved.

### <Method of manufacturing soap composite particles>

The soap composite particles of the present embodiment can be produced, for example, through a step of pulverizing a saturated higher fatty acid salt, a step of externally adding an inorganic oxide to core particles, and a step of spheroidizing the core particles. The spheroidization step can be carried out before or after the external addition step.

In the case where the spheroidization step is carried out before the external addition step, the production method preferably comprises a pulverization step of pulverizing a solid of the saturated higher fatty acid salt, a spheroidization step of spheroidizing the particles obtained in the pulverization step, and an external addition step of externally adding an inorganic oxide to the core particles obtained in the spheroidization step.

In the case where the spheroidization step is carried out after the external addition step, the production method preferably comprises a pulverization step of pulverizing a solid of the saturated higher fatty acid salt, an external addition step of externally adding an inorganic oxide to the particles obtained in the pulverization step, and a spheroidization step of spheroidizing the particles obtained in the external addition step.

When an inorganic oxide is added internally to the core particles, it is preferable to have a kneading step of melting and kneading a mixed powder of a saturated higher fatty acid salt and an inorganic oxide before the pulverizing step. When no inorganic oxide is added internally to the core particles, it is preferable to heat and melt the saturated higher fatty acid salt to obtain a solid product of the saturated higher fatty acid salt.

When an inorganic oxide is added internally to the core particles, the kneading step may be preceded by a measuring step of measuring the saturated higher fatty acid salt and the inorganic oxide to be used in preparing the core particles, and a mixing step of stirring and mixing the measured saturated higher fatty acid salt and the inorganic oxide. The particles can be mixed using any mixing device, including, but not limited to, a double cone mixer, a V-type mixer, a drum mixer, a super mixer, a Henschel mixer, a Nauta mixer, and the like.

Although it is possible to mix some of the raw materials during the kneading step, it is preferable to premix at least some of the raw materials before the kneading step. By carrying out the pre-mixing, it is convenient to control the composition of the saturated higher fatty acid salt and inorganic oxide, the size and shape of the particles, and the like.

The kneading step is preferably carried out under conditions in which the saturated higher fatty acid salt is melted or softened by heating. As a result, the inorganic oxide mixed with the saturated higher fatty acid salt is added internally to the saturated higher fatty acid salt. The kneader may be a batch type kneader or a continuous type kneader. The kneading machine is not particularly limited, but examples thereof include an open roll, a kneader, a pressure kneader, a Banbury mixer, a single-screw extruder, and a twin-screw extruder. The heating temperature in the kneading step can be appropriately set depending on the melting point etc. of the saturated higher fatty acid salt, and may be, for example, about 80 to 250°C, about 100 to 200°C, or 100 to 140°C.

When a kneading step of the saturated higher fatty acid salt and the inorganic oxide or a melting step of the saturated higher fatty acid salt is carried out prior to the pulverization step, it is preferable to carry out a cooling step in which the kneaded product obtained in the kneading step or the melted product obtained in the melting step is cooled. The residual heat may be absorbed by a cooling member in contact with the kneaded or melted material, for example, by passing the molten kneaded or melted material through a cooling roll. The cooling member such as the cooling roll may contain a refrigerant such as cooling water inside. In the cooling step, heat can be dissipated from the kneaded material by air cooling, blowing air, or the like.

The pulverization step may be carried out in multiple stages, such as a coarse pulverization step and a fine pulverization step. The device used for coarsely crushing the particles is not particularly limited, but a crusher, hammer mill, feather mill, cutter mill, etc. can be used. The device used for finely pulverizing the particles is not particularly limited, but a jet mill, a counter jet mill, a high-speed rotor rotary mill, etc. can be used.

When the solid is pulverized to a desired particle size, the particle size may be adjusted by a classification step, if necessary. In the classification step, for example, an elbow jet classifier of an inertial classification type, a Microplex of a centrifugal classification type, an airflow type classifier, or the like can be used. Particles that are coarser than the desired particle size may be returned to the pulverization step for further pulverizing. Particles finer than the desired particle size may be recycled by returning them to the kneading or melting step, depending on the composition.

The spheroidization step is a step in which particles are spheroidized by heating. The spheroidizing device is not particularly limited, but examples thereof include a device that mechanically adjusts particle shape, such as an impact spheroidizing device; a device that adjusts particle shape by binder dissolution and solvent removal, such as a spray dryer; and a device that adjusts particle shape by heating in a medium or using hot air. When the spheroidization step is carried out before the external addition step, spherical core particles can be obtained by the spheroidization step. When the spheroidization step is carried out after the external addition step, soap composite particles having spherical core particles can be obtained by the spheroidization step. If necessary, a classification step can be carried out after the spheroidization step.

In the external addition step, the inorganic oxide is mixed with the core particles to externally add the inorganic oxide to the core particles. In the step of externally adding the inorganic oxide, a mixing device such as a super mixer or a Henschel mixer may be used, or a powder processing device such as a Mechanomill may be used.

### <Soap composite particles>

The soap composite particles of the embodiment have the functionality of the saturated higher fatty acid salt as well as the functionality of the inorganic oxide added internally or externally, and therefore can be used as a variety of functional materials. The uses of the soap composite particles are not particularly limited, but examples thereof include use as a feel improver, lubricant, release agent, anti-caking agent, cosmetic raw material, colorant, processing aid, dispersant, additive, etc.

### <Method for analyzing soap composite particles>

As described above, the soap composite particle of the embodiment has a core particle containing a saturated higher fatty acid salt and an inorganic oxide added externally to the core particle. Since the core particles are mainly composed of saturated higher fatty acid salts, it is possible to separate the saturated higher fatty acid salts from the inorganic oxides. This makes it possible to evaluate the amount of internal addition and the amount of external addition even if the inorganic oxide internally added to the core particle and the inorganic oxide externally added to the core particle are the same substance.

A specific example of a method for separating the core particles from the external additive (externally added inorganic oxide) is a method in which a pre-weighed amount of soap composite particle powder is added to a dispersion medium, and the resulting dispersion is then irradiated with ultrasonic waves by inserting the probe of an ultrasonic homogenizer. This allows the external additives that have fallen off the core particles to be suspended in the dispersion medium. The core particles are allowed to settle by standing or centrifuging, and then the supernatant liquid in which the external additives are suspended can be extracted and separated by a liquid separation operation or the like.

The dispersion medium is preferably an aqueous solution prepared by dissolving a surfactant in water in order to prevent the powder from agglomerating. Since the saturated higher fatty acid salt is insoluble in water, the inorganic oxide added internally thereto can remain enclosed in the core particles.

The amount of core particles from which the external additives have been separated can be determined by repeatedly washing the sediment with a solvent and filtering it using filter paper capable of collecting the core particles, and then drying the sediment and weighing it. The collection performance of the filter paper is preferably such that it can retain particles of 1 µm or larger. The amount of the external additive may be calculated by subtracting the mass of the core particles obtained by weighing from the mass of the soap composite particles.

The components and composition of the external additives contained in the supernatant can be analyzed by, for example, X-ray fluorescence analysis (XRF) of the supernatant.

The components and composition of the inorganic oxide added internally to the core particles can be analyzed, for example, by X-ray fluorescence analysis (XRF) of the core particles obtained as a precipitate.

### <Cosmetic containing soap composite particles>

The cosmetic of the present embodiment contains the soap composite particles. Examples of cosmetics containing soap composite particles include makeup cosmetics such as foundation, liquid foundation, eye shadow, rouge, face powder, concealer, eyebrow powder, and highlighter; cleansing agents such as cleansing agents and facial washes; skin care cosmetics such as massage cream, moisturizing cream, and emulsion; body care cosmetics such as bath additives, sunscreen, sunscreen cream, and deodorant spray; basic cosmetics such as makeup base; and hair care cosmetics such as shampoo, conditioner, hair liquid, and hair color. These cosmetics can be prepared by mixing and processing various raw materials in proportions suitable for the intended use.

The cosmetic may contain the soap composite particles in an amount of 0.5% by mass to 60% by mass relative to the total amount of the cosmetic. Preferably, the soap composite particles can be contained in the range of 1% by mass to 40% by mass, more preferably 2% by mass to 30% by mass, based on the total amount of the cosmetic. If the content of soap composite particles is less than 0.5% by mass, it is difficult to feel the effect of improving the skin feel when using the cosmetic product, and if it exceeds 60% by mass, it is not possible to expect an effect commensurate with the amount blended, and problems may arise in maintaining the quality and stability of the cosmetic product.

In addition to the soap composite particles, various ingredients that have been conventionally used in cosmetics can be blended into the cosmetics. Examples of the components include pigments, surface-treated pigments, ultraviolet absorbers, physiologically active ingredients, oils, surfactants, fluorine compounds, resins, thickeners, preservatives, fragrances, moisturizers, salts, solvents, antioxidants, chelating agents, neutralizing agents, pH adjusters, and insect repellents. These ingredients can be blended in amounts that do not impair the effects of the cosmetic composition of the present invention.

The cosmetic may contain a paraffin-based solvent such as isododecane. In the soap composite particles, the inorganic oxide particles are less likely to fall off due to the hydrophobic surface of the core particle, and the functionality of the metal soap particles is excellent in sustainability. Furthermore, when the surface of the inorganic oxide is subjected to a hydrophobic treatment, the affinity of the inorganic oxide to the surface of the core particle is increased, and the falling off of the inorganic oxide particles is further suppressed.

The present invention has been described above based on preferred embodiments, but the present invention is not limited to the above-described embodiments, and various modifications are possible within the scope of the gist of the present invention.

### Examples

Examples of the present invention will be described below, but the present invention is not limited to these examples.

### <Production of soap composite particles>

By carrying out the following steps, soap composite particles were produced in which inorganic oxides were externally added to core particles containing saturated higher fatty acid salts and inorganic oxides.

### (Mixing step)

As shown in the "saturated higher fatty acid salt" and "internal inorganic oxide" columns of Tables 1 to 4, the saturated higher fatty acid salt and the inorganic oxide were each weighed, placed in a 20 L Henschel mixer (FM20 manufactured by Nippon Coke and Engineering Co., Ltd.), and mixed by stirring to obtain a mixed powder.

### (Kneading step)

The mixed powder obtained in the mixing step was heated to 100 to 140° C. and melt-kneaded in a twin-screw kneader (PCM-30 manufactured by Ikegai Corporation). The molten mixture discharged from the kneader was passed through cooled pressure rolls to obtain a plate-shaped solid mixture.

### (Crushing step)

The plate-like solid kneaded product obtained in the mixing step was coarsely pulverized in a hammer mill to obtain coarsely pulverized products of 0.5 to 3.0 mm. The coarsely ground material was finely ground in a jet mill (ULTRA SONIC JET MILL I-2 manufactured by Nippon Pneumatic Mfg. Co., Ltd.) to obtain a finely ground material having an average particle size by volume of 3 to 20 µm.

### (Classification step)

The finely pulverized material obtained in the pulverization step was passed through an air classifier (DS2UR manufactured by Nippon Pneumatic Mfg. Co., Ltd.) to remove particles of 3 µm or less.

### (Spheroidizing step)

The classified powder obtained in the classification step was stirred for 20 minutes in a jacketed Henschel mixer 10 L (FM10 manufactured by Nippon Coke) through which hot water heated to 60 to 90°C was circulated, thereby forming spherical particles.

### (External addition step)

To the spherical core particles obtained in the spheroidization step, inorganic oxides were added externally as shown in "Externally added inorganic oxides" in Tables 1 to 4 to obtain soap composite particles.

### <Core particle structure>

The spherical core particles obtained in the spheroidization step were used as samples, and the structure of the core particles was measured by the following method. It is also possible to use the core particles separated from the external additives using the above-mentioned method for analyzing soap composite particles as a sample.

### (Circularity)

The circularity of the core particles was measured using a flow particle image analyzer.

### (Volume-based median diameter)

The median diameter (D50) was determined by measuring the volume distribution using a Coulter counter, utilizing the electrical change that occurs when the core particles pass through an aperture tube.

### <How to evaluate skin feel>

A quantity of the soap composite particle powder approximately equal to one ear-pick scoop was placed on the wrist of one arm, and the powder was gently rolled over the skin with the middle finger of the other hand in a circular motion with a diameter of 4 cm for 15 seconds, and the feel was evaluated using the middle finger and wrist. Four evaluation panelists (two adult males and two adult females) classified the sensation of use into each category and gave scores to each category to perform a sensory evaluation. The evaluation items and evaluation criteria are as follows:

### (Evaluation items for skin feel)

Spreadability: Whether the powder spreads well on the skin when applied.

Adhesion strength: Whether the powder fills into the pores well when applied.

Smoothness: When applying powder, does it feel smooth on the skin after application?

Lightness: When applying the powder, does it feel light after application?

Lasting power: Whether the makeup lasts well when you rub it with your fingers after applying the powder.

Softness: When applying powder, is the powder soft enough after application?

### (Evaluation criteria for skin feel)

5: Absolutely Excellent.
4: Excellent.
3: Normal.
2: Inferior.
1: Very poor.

### <Expression of function>

The expression of the functions of the inorganic oxides was evaluated for each type of inorganic oxide according to the following evaluation criteria.

### (Evaluation criteria for the expression of function)

5: The function of the inorganic oxide is expressed.
3: The function of the inorganic oxide is slightly expressed.
1: The function of the inorganic oxide is not expressed.

### <Function sustainability>

9.5 g of a volatile organic solvent used in cosmetics was placed in a sample bottle, 0.5 g of soap composite particle powder was added, the bottle was capped, and the bottle was placed on a vibration table and stirred, and then left to stand in a room at 20°C for 24 hours. The humidity was set within the range of normal humidity (45 to 85% RH). The particles were observed under an optical microscope to see if their shape had changed from the shape before immersion in the solvent. The sustainability of function was evaluated according to the following criteria.

### (Evaluation criteria for sustainability of function)

5: No change in the shape of the soap composite particles.
3: Partial change in the shape of the soap composite particles.
1: The shape of the soap composite particles was changed.

### <Overall evaluation>

The overall evaluation was based on the total score obtained from the three items of (1) skin feel, (2) expression of function, and (3) sustainability of function, and was evaluated according to the following evaluation criteria.

### (Evaluation criteria for overall evaluation)

+++: Total score was 15.
++: Total score was 13 or 14.
+: Total score was 11 or 12.
-: Total score was less than 11.

### <Evaluation results>

The evaluation results are shown in Tables 1 to 4. In the "Saturated higher fatty acid salts" column, fatty acid salts are displayed by combining the name of the fatty acid with the element symbol of the metal, for example, "Ca stearic acid" represents "calcium stearate". In some comparative examples, unsaturated fatty acid salts were used.

Stearic acid (18 carbon atoms), lauric acid (12 carbon atoms), palmitic acid (16 carbon atoms), caprylic acid (8 carbon atoms), and behenic acid (22 carbon atoms) are straight-chain saturated fatty acids. Oleic acid (18 carbon atoms, 1 double bond) and linoleic acid (18 carbon atoms, 2 double bonds) are straight-chain unsaturated fatty acids.

The weight parts in Tables 1 to 4 were set so that the total amount of saturated higher fatty acid salt and inorganic oxide added was 100 weight parts. Therefore, the numerical value of the amount (parts by weight) of the inorganic oxide added internally is equal to the numerical value of the content (% by weight) of the inorganic oxide in the core particles.

In the "Hydrophobic Treatment" column of Tables 1 to 4, the inorganic oxide that was not hydrophobic treated is indicated as "None," the silane coupling treatment is indicated as "SiCp," and the silicone oil treatment is indicated as "SiOi."

**[Table 1]**

| | | Ex.1 | Ex.2 | Ex.3 | Ex.4 | Ex.5 | Ex.6 | Ex.7 | Ex.8 |
|---|---|---|---|---|---|---|---|---|---|
| Internally added fatty acid salt (parts by weight) | Stearic acid Ca | 93 | | | 93 | 93 | 93 | 93 | 93 |
| | Lauric acid Zn | | 93 | | | | | | |
| | Palmitic acid Na | | | 93 | | | | | |
| | Caprylic acid Na | | | | | | | | |
| | Behenic acid Na | | | | | | | | |
| | Oleic acid Na | | | | | | | | |
| | Linoleic acid Ca | | | | | | | | |
| Internally added inorganic oxide (parts by weight) | Zinc oxide | 7 | 7 | 7 | | | | | |
| | Magnesium oxide | | | | 7 | | | | |
| | Calcium oxide | | | | | 7 | | | |
| | Aluminum oxide | | | | | | 7 | | |
| | Titanium dioxide | | | | | | | 7 | |
| | Iron oxide | | | | | | | | 7 |
| Core particle | Total (parts by weight) | 100 | 100 | 100 | 100 | 100 | 100 | 100 | 100 |
| Externally added inorganic oxide (parts by weight) | Zinc oxide | 7 | 7 | 7 | | | | | |
| | Magnesium oxide | | | | 7 | | | | |
| | Calcium oxide | | | | | 7 | | | |
| | Aluminum oxide | | | | | | 7 | | |
| | Titanium dioxide | | | | | | | 7 | |
| | Iron oxide | | | | | | | | 7 |
| Core particle structure | Circularity | 0.91 | 0.91 | 0.91 | 0.91 | 0.91 | 0.91 | 0.91 | 0.91 |
| | D50(µm) | 9 | 9 | 9 | 9 | 9 | 9 | 9 | 9 |
| Characteristic of inorganic oxide | Average primary particle size (µm) | 0.3 | 0.3 | 0.3 | 0.2 | 0.5 | 0.4 | 0.02 | 1.0 |
| | Hydrophobic treatment | SiCp | SiCp | SiCp | SiCp | SiCp | SiCp | SiCp | SiCp |
| Evaluation | Skin feel | 5 | 5 | 4 | 4 | 4 | 4 | 4 | 4 |
| | Expression of function | 5 | 5 | 5 | 5 | 5 | 5 | 5 | 5 |
| | Sustainability of function | 5 | 5 | 5 | 5 | 5 | 5 | 5 | 5 |
| | Total score | 15 | 15 | 14 | 14 | 14 | 14 | 14 | 14 |
| | Overall evaluation | +++ | +++ | ++ | ++ | ++ | ++ | ++ | ++ |

**[Table 2]**

| | | Ex.9 | Comp. Ex.1 | Comp. Ex.2 | Ex.10 | Comp. Ex.3 | Comp. Ex.4 | Ex. 11 | Ex.12 |
|---|---|---|---|---|---|---|---|---|---|
| Internally added fatty acid salt (parts by weight) | Stearic acid Ca | 88 | 84 | 93 | 93 | | | | |
| | Lauric acid Zn | | | | | | | | |
| | Palmitic acid Na | | | | | | | | |
| | Caprylic acid Na | | | | | | | 93 | |
| | Behenic acid Na | | | | | | | | 93 |
| | Oleic acid Na | | | | | 93 | | | |
| | Linoleic acid Ca | | | | | | 93 | | |
| Internally added inorganic oxide (parts by weight) | Zinc oxide | 12 | 16 | 7 | 7 | 7 | 7 | 7 | 7 |
| | Magnesium oxide | | | | | | | | |
| | Calcium oxide | | | | | | | | |
| | Aluminum oxide | | | | | | | | |
| | Titanium dioxide | | | | | | | | |
| | Iron oxide | | | | | | | | |
| Core particle | Total (parts by weight) | 100 | 100 | 100 | 100 | 100 | 100 | 100 | 100 |
| Externally added inorganic oxide (parts by weight) | Zinc oxide | 7 | 7 | 7 | 7 | 7 | 75 | 7 | 7 |
| | Magnesium oxide | | | | | | | | |
| | Calcium oxide | | | | | | | | |
| | Aluminum oxide | | | | | | | | |
| | Titanium dioxide | | | | | | | | |
| | Iron oxide | | | | | | | | |
| Core particle structure | Circularity | 0.91 | 0.91 | 0.7 | 0.85 | 0.91 | 0.91 | 0.91 | 0.91 |
| | D50(µm) | 9 | 9 | 9 | 9 | 9 | 9 | 9 | 9 |
| Characteristic of inorganic oxide | Average primary particle size (µm) | 0.3 | 0.3 | 0.3 | 0.3 | 0.3 | 0.3 | 0.3 | 0.3 |
| | Hydrophobic treatment | SiCp | SiCp | SiCp | SiCp | SiCp | SiCp | SiCp | SiCp |
| Evaluation | Skin feel | 4 | 4 | 3 | 3 | 2 | 2 | 3 | 2 |
| | Expression of function | 5 | 5 | 5 | 5 | 3 | 3 | 5 | 5 |
| | Sustainability of function | 5 | 1 | 1 | 5 | 5 | 5 | 3 | 5 |
| | Total score | 14 | 10 | 9 | 13 | 10 | 10 | 11 | 12 |
| | Overall evaluation | ++ | - | - | ++ | - | - | + | + |

**[Table 3]**

| | | Ex.13 | Ex.14 | Ex.15 | Ex.16 | Ex.17 | Ex.18 |
|---|---|---|---|---|---|---|---|
| Internally added fatty acid salt (parts by weight) | Stearic acid Ca | 93 | 93 | 93 | 93 | 93 | 93 |
| | Lauric acid Zn | | | | | | |
| | Palmitic acid Na | | | | | | |
| | Caprylic acid Na | | | | | | |
| | Behenic acid Na | | | | | | |
| | Oleic acid Na | | | | | | |
| | Linoleic acid Ca | | | | | | |
| Internally added inorganic oxide (parts by weight) | Zinc oxide | 7 | 7 | 7 | 7 | 7 | 7 |
| | Magnesium oxide | | | | | | |
| | Calcium oxide | | | | | | |
| | Aluminum oxide | | | | | | |
| | Titanium dioxide | | | | | | |
| | Iron oxide | | | | | | |
| Core particle | Total (parts by weight) | 100 | 100 | 100 | 100 | 100 | 100 |
| Externally added inorganic oxide (parts by weight) | Zinc oxide | 0.005 | 0.03 | 12 | 18 | 7 | 7 |
| | Magnesium oxide | | | | | | |
| | Calcium oxide | | | | | | |
| | Aluminum oxide | | | | | | |
| | Titanium dioxide | | | | | | |
| | Iron oxide | | | | | | |
| Core particle structure | Circularity | 0.91 | 0.91 | 0.91 | 0.91 | 0.91 | 0.91 |
| | D50(µm) | 9 | 9 | 9 | 9 | 9 | 9 |
| Characteristic of inorganic oxide | Average primary particle size (µm) | 0.3 | 0.3 | 0.3 | 0.3 | 0.3 | 0.3 |
| | Hydrophobic treatment | SiCp | SiCp | SiCp | SiCp | None | SiOi |
| Evaluation | Skin feel | 4 | 3 | 3 | 1 | 2 | 4 |
| | Expression of function | 3 | 5 | 5 | 5 | 5 | 5 |
| | Sustainability of function | 5 | 5 | 5 | 5 | 5 | 5 |
| | Total score | 12 | 13 | 13 | 11 | 12 | 14 |
| | Overall evaluation | + | ++ | ++ | + | + | ++ |

**[Table 4]**

| | | Ex.19 | Ex.20 | Ex.21 | Ex.22 | Ex.23 | Ex.24 | Ex.25 | Ex.26 |
|---|---|---|---|---|---|---|---|---|---|
| Internally added fatty acid salt (parts by weight) | Stearic acid Ca | 93 | 93 | 93 | 93 | 93 | 93 | 93 | 93 |
| | Lauric acid Zn | | | | | | | | |
| | Palmitic acid Na | | | | | | | | |
| | Caprylic acid Na | | | | | | | | |
| | Behenic acid Na | | | | | | | | |
| | Oleic acid Na | | | | | | | | |
| | Linoleic acid Ca | | | | | | | | |
| Internally added inorganic oxide (parts by weight) | Zinc oxide | 7 | 7 | 7 | 7 | 7 | 7 | 7 | 7 |
| | Magnesium oxide | | | | | | | | |
| | Calcium oxide | | | | | | | | |
| | Aluminum oxide | | | | | | | | |
| | Titanium dioxide | | | | | | | | |
| | Iron oxide | | | | | | | | |
| Core particle | Total (parts by weight) | 100 | 100 | 100 | 100 | 100 | 100 | 100 | 100 |
| Externally added | Zinc oxide | 7 | 7 | 7 | 7 | 7 | 7 | 7 | 7 |
| inorganic oxide (parts by weight) | Magnesium oxide | | | | | | | | |
| | Calcium oxide | | | | | | | | |
| | Aluminum oxide | | | | | | | | |
| | Titanium dioxide | | | | | | | | |
| | Iron oxide | | | | | | | | |
| Core particle structure | Circularity | 0.91 | 0.91 | 0.91 | 0.91 | 0.91 | 0.91 | 0.91 | 0.91 |
| | D50(µm) | 9 | 9 | 9 | 9 | 2 | 4 | 17 | 25 |
| Characteristic of inorganic oxide | Average primary particle size (µm) | 0.005 | 0.1 | 0.8 | 1.3 | 0.3 | 0.3 | 0.3 | 0.3 |
| | Hydrophobic treatment | SiCp | SiCp | SiCp | SiCp | SiCp | SiCp | SiCp | SiCp |
| Evaluation | Skin feel | 2 | 4 | 4 | 2 | 2 | 3 | 3 | 2 |
| | Expression of function | 5 | 5 | 5 | 5 | 5 | 5 | 5 | 5 |
| | Sustainability of function | 5 | 5 | 5 | 5 | 5 | 5 | 5 | 5 |
| | Total score | 12 | 14 | 14 | 12 | 12 | 13 | 13 | 12 |
| | Overall evaluation | + | ++ | ++ | + | + | ++ | ++ | + |

In Examples 1 to 26, the overall evaluation was +++, ++, or +, and it was possible to achieve both an improvement in the feel on the skin and the sustainability of the function of the soap composite particles.

In Comparative Examples 1 to 4, the overall evaluation was -.

In Comparative Example 1, the content of the specific metal oxide in the core particles was more than 10 mass %, and the sustainability of function was 1.

In Comparative Example 2, the circularity was less than 0.80, and the sustainability of function was 1.

In Comparative Examples 3 and 4, unsaturated fatty acid salts were used instead of saturated higher fatty acid salts, but the evaluation of skin feel was low.

### INDUSTRIAL APPLICABILITY

The soap composite particles of the present invention can achieve both an improvement in skin feel and sustainability of the inorganic oxide function, and can be used in the production of cosmetics.

## Claims

1. Soap composite particles comprising a core particle containing a saturated higher fatty acid salt and an inorganic oxide externally added to the core particle, wherein,
the total content of Li₂O, Na₂O, Al₂O₃, MgO, CaO, ZnO, BaO, TiO₂, SiO₂, Fe₃O₄, Fe₂O₃, and FeO in the core particle is 0 to 15 mass%, and
the shape of the core particle is spherical with a circularity of 0.80 to 1.00.

2. The soap composite particles according to claim 1, wherein the saturated higher fatty acid salt has a carbon number in the range of 10 to 20.

3. The soap composite particles according to claim 1, wherein a metal forming the saturated higher fatty acid salt is at least one kind selected from Li, Na, Al, Mg, Ca, Zn, and Ba.

4. The soap composite particles according to claim 1, wherein the amount of the inorganic oxide added externally is 0.01 to 15 parts by weight relative to 100 parts by weight of the core particles.

5. The soap composite particles according to claim 1, wherein the inorganic oxide is one or more kind selected from zinc oxide, magnesium oxide, titanium dioxide, mica, talc, kaolin, sericite, silica, iron oxide, alumina, zirconia, and zeolite.

6. The soap composite particles according to claim 1, wherein the inorganic oxide is subjected to a hydrophobic treatment.

7. The soap composite particles according to claim 6, wherein the hydrophobic treatment is any one of a silane coupling treatment, a silicone oil treatment, a long-chain alkylsilane treatment, a fatty acid salt treatment, a long-chain alkane treatment, a long-chain alkene treatment, and a long-chain alkyne treatment.

8. The soap composite particles according to claim 1, wherein the inorganic oxide has an average primary particle size of 0.01 to 1.0 µm.

9. The soap composite particles according to claim 1, wherein the volume-based median diameter of the core particle is greater than 3 µm and less than 20 µm.

10. A method for producing the soap composite particles according to claim 1, comprising:
a pulverization step of pulverizing a solid of the saturated higher fatty acid salt;
a spheroidization step of spheroidizing the particles obtained in the pulverization step; and
an external addition step of externally adding an inorganic oxide to the core particles obtained in the spheroidization step.

11. A method for producing the soap composite particles according to claim 1, comprising:
a pulverization step of pulverizing a solid of the saturated higher fatty acid salt;
an external addition step of externally adding an inorganic oxide to the particles obtained in the pulverization step; and
a spheroidization step of spheroidizing the particles obtained in the external addition step.

12. A cosmetic **characterized by** comprising the soap composite particles according to any one of claims 1 to 9.
